(19) **Europäisches Patentamt**
**European Patent Office**
**Office européen des brevets**

(11) **EP 3 644 921 B1**

(12) **EUROPEAN PATENT SPECIFICATION**

(45) Date of publication and mention
of the grant of the patent:
**07.04.2021 Bulletin 2021/14**

(21) Application number: **18743313.1**

(22) Date of filing: **29.06.2018**

(51) Int Cl.:
*A61F 13/476* (2006.01)      *A61F 13/56* (2006.01)
*A61F 13/47* (2006.01)

(86) International application number:
**PCT/US2018/040164**

(87) International publication number:
**WO 2019/006224 (03.01.2019 Gazette 2019/01)**

(54) **FEMININE HYGIENE ARTICLE HAVING ADHESIVE SIDE EXTENSIONS WITH ENHANCED LONGITUDINAL FLEXURE AND STRUCTURAL INTEGRITY**

ARTIKEL FÜR DIE WEIBLICHE HYGIENE MIT HAFTSEITENVERLÄNGERUNGEN MIT VERBESSERTER LÄNGSBIEGUNG UND STRUKTURINTEGRITÄT

ARTICLE D'HYGIÈNE FÉMININE COMPORTANT DES EXTENSIONS LATÉRALES ADHÉSIVES QUI PRÉSENTENT UNE FLEXION LONGITUDINALE ET UNE INTÉGRITÉ STRUCTURELLE AMÉLIORÉES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.06.2017 US 201715639279**

(43) Date of publication of application:
**06.05.2020 Bulletin 2020/19**

(73) Proprietor: **The Procter & Gamble Company Cincinnati, OH 45202 (US)**

(72) Inventors:
• **SHEEHAN, Astrid Annette**
**Cincinnati**
**Ohio 45202 (US)**

• **LUDHER, Callida Williams**
**Cincinnati**
**Ohio 45202 (US)**

(74) Representative: **P&G Patent Germany**
**Procter & Gamble Service GmbH**
**Sulzbacher Straße 40**
**65824 Schwalbach am Taunus (DE)**

(56) References cited:
**WO-A1-2017/115761      US-A1- 2005 124 958**
**US-A1- 2016 213 526**

Remarks:
The file contains technical information submitted after the application was filed and not included in this specification

EP 3 644 921 B1

**Description**

BACKGROUND OF THE INVENTION

A number of types of currently available feminine hygiene pads have adhesive side extensions (often known as "wings") for wrapping over and around the inside leg opening edges of the user's underpants and adhering to the underside thereof, for purposes of maintaining the pad in position within the underpants and providing enhanced protection against soiling of the underpants proximate the leg openings. These may be deemed by users to have a shortcoming. The side extensions are typically formed of integral extensions of topsheet and/or backsheet material, and have a patch or layer of exposable adhesive deposited thereon for purposes of adhering the side extensions to the outer/underside surface of the underpants. Being formed of topsheet and/or backsheet material, the side extensions are typically relatively low basis weight, low caliper materials. As a consequence, the side extensions are typically very thin and pliable, and may be susceptible to being mis-folded along unintended fold lines, mis-applied, crumpled, bunched and/or stuck to themselves, in the processes of unpackaging and applying the product to the inside of underpants. This can lead to loss of utility of the side extensions and even of the entire pad, and resulting user frustration and dissatisfaction with the product. Any improvement which would reduce or eliminate such occurrences while preserving the utility of the side extensions and the comfortableness of the product in use would benefit the users/consumers and provide competitive advantage to the manufacturer.

US 2016/213526 A1 discloses an absorbent article including reinforcement sheets in the wings.

The present invention relates to an absorbent feminine hygiene article as defined in claims 1-14.

BRIEF DESCRIPTION OF THE DRAWINGS

FIG. 1 is a schematic plan view of a feminine hygiene pad.
FIGS. 2A-2C are schematic lateral cross sections of alternative examples of a feminine hygiene pad.
Fig. 3 is a schematic plan view of an absorbent core of a feminine hygiene product, illustrating the method herein for identifying left and right core ¼ length chord lines.

DETAILED DESCRIPTION OF THE INVENTION

Definitions

"Inboard," with respect to the location of a first feature with respect to the location of a second feature of a feminine hygiene pad, means that the first feature lies closer to the longitudinal axis of the pad than the second feature, in plan.

"Outboard," with respect to the location of a first feature with respect to the location of a second feature of a feminine hygiene pad, means that the first feature lies farther from the longitudinal axis of the pad than the second feature, in plan.

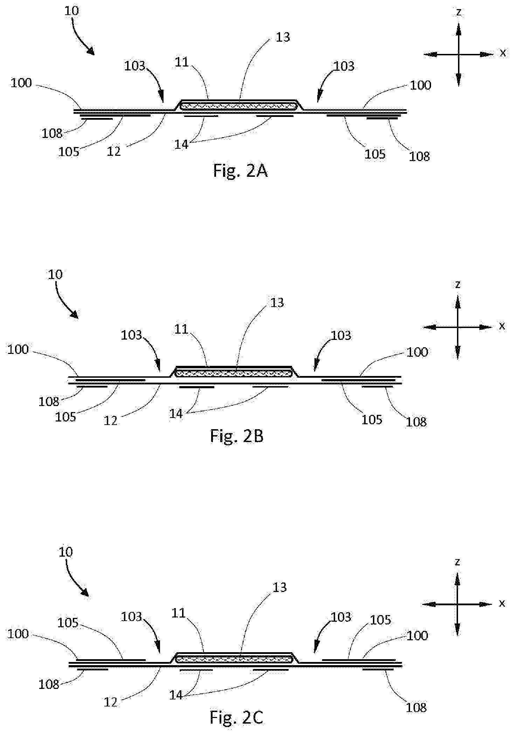

"Outward-facing" or "garment-facing," with respect to a surface or web component of a feminine hygiene pad, means the surface or web component on the outside of the pad, or on the surface facing away from the wearer, when the pad is in use. "Wearer-facing," with respect to a surface or web component of a feminine hygiene pad, means the surface or web component on the inside of the pad, on the surface facing toward the wearer, when the pad is in use. (With respect to side extensions, the outward-facing or garment facing surfaces are identified with the side extensions extended laterally outward, as they are shown in Figs. 2A-2C, prior to folding or wrapping. In Figs. 2A-2C, all outward/garment-facing surfaces are at the bottoms in the figures, and all wearer-facing surfaces are at the tops of the figures.)

"Lateral" and forms thereof refer to a direction approximately perpendicular to the longitudinal axis of the feminine hygiene pad in plan.

"Longitudinal" and forms thereof refer to a direction approximately parallel to the longitudinal axis of the feminine hygiene pad in plan.

"x-direction" means a direction perpendicular to the longitudinal axis of the feminine hygiene pad in plan.

"y-direction" means a direction parallel to the longitudinal axis of the feminine hygiene pad in plan.

"z-direction" means a direction perpendicular to the plane defined by the x- and y- axes.

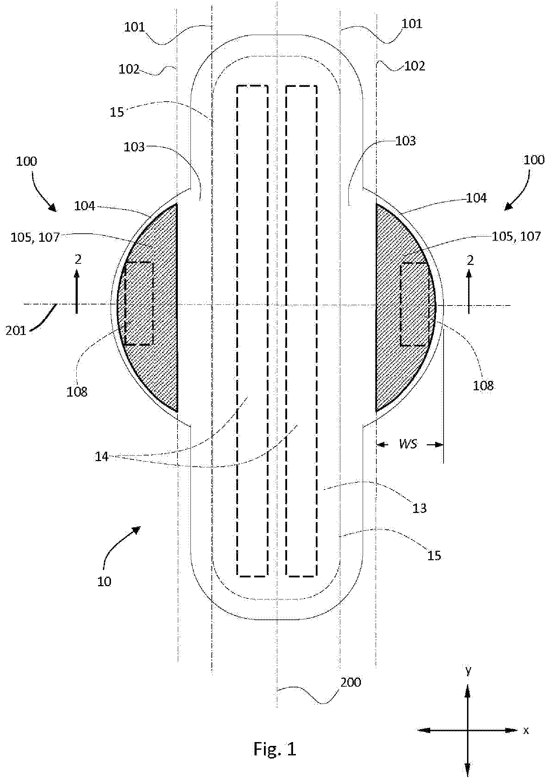

Referring to Figs. 1 and 2A-2C, a feminine hygiene pad 10 of the type contemplated herein typically includes a topsheet 11, a backsheet 12, and an absorbent core 13 disposed therebetween. Examples of feminine hygiene pads contemplated include pads adapted for wear at the crotch region inside the user's underpants, marketed and sold as useful for receiving, containing and absorbing exudates resulting from menstruation, and/or for receiving, containing and absorbing urine for users experiencing mild urinary incontinence, including the relatively thin, low caliper pads sometimes known as panty shields, light-duty pads and the like.

The topsheet 11 may be formed from any generally soft, compliant, and porous material which is comfortable against human skin and through which bodily exudates can pass. Examples of materials suitable for forming topsheets include nonwovens and apertured polymeric films, known and disclosed in the arts of designing and manufacturing feminine hygiene pads, disposable diapers and disposable absorbent pants.

Backsheets are generally configured to hinder or prevent bodily exudates from passing entirely through the pad and soiling a user's underwear or outer garments. Material forming the backsheet 12 may be designed, selected and/or assembled so as to be substantially liquid impermeable under normal conditions of use, and may be formed from polymer films or film/nonwoven laminates. Material forming the backsheet 12 may be designed, selected and or assembled so as to be vapor permeable while being liquid impermeable under normal conditions of use, to help render the article more breathable. Examples of vapor permeable materials include microporous films, apertured formed films, and nonwovens, also known and disclosed in the arts of feminine hygiene pads, disposable diapers and disposable absorbent pants. Any of such materials may include an additive (*e.g.*, wax or surfactant) or treatment to increase their hydrophobicity or hydrophilicity.

The absorbent core 13 may be formed from any of the materials known to those of ordinary skill in the arts of design and manufacture of feminine hygiene pads, disposable diapers and disposable absorbent pants. Examples of such materials include, but are not limited to, plies of creped cellulose wadding, fluffed cellulose fibers, wood pulp fibers (sometimes known as "airfelt"), a mass or batt of fibers, airlaid webs or fibers, a web of polymeric fibers, and a blend of polymeric fibers. The absorbent core may also comprise high capacity materials such as polyacrylate particles (often referred to as "superabsorbents" or absorbent gelling materials (AGM)), and high internal phase emulsion (HIPE) foams such as, but not limited to, those disclosed in U.S. Pat. Nos. 5,550,167; 5,387,207; 5,352,711; and 5,331,015.

Optionally, one or more intermediate layers between the topsheet and backsheet may be included. In one configuration, an intermediate layer may be disposed between the topsheet and the absorbent core. Such layers are included in many known absorbent article designs and may be identified as "secondary topsheets," "surge layers," "acquisition layers," "distribution layers," "transport layers," "wicking layers" and the like. An intermediate layer may serve to facilitate rapid acquisition of bodily exudates entering through the topsheet and distribution of the same across and into the absorbent core, and/or to temporarily hold bodily exudates as a means for managing large loading volumes or fast loading rates. Non-limiting examples of surge layers are disclosed in U.S. Pat. Nos. 5,843,063 and 5,879,343. In another configuration, an intermediate layer may be disposed between the absorbent core and the backsheet. In this location, an intermediate layer may provide any number of benefits including, for example, structural integrity, flexibility, body shaping, and bodily exudates management.

Absorbent articles of the present invention may be individually wrapped. The wrapping material may help provide cleanliness and discretion when they are carried outside of the home. The wrappers generally at least partially enclose the absorbent article, and preferably, substantially completely enclose the article. In some examples, the wrapper is configured to accept a used article to facilitate disposal of the same. The wrappers may be constructed from numerous materials, including, for example, polymeric films, fibrous materials (including nonwovens and wovens), paper, card stock, and combinations thereof. In one particular example, the wrappers are constructed from flexible, polymeric films. The polymeric films may be based on polyethylene, polypropylene, polyester, nylon, polyvinyl alcohol, or blends of the same. One example of a material is a 32 gauge polypropylene film. The materials may be a single layer or more than one layer. The wrappers may be formed from a single feedstock of material that is manipulated into a container, such as by folding, or may be formed from multiple feedstocks that are joined together to ultimately form the finished wrapper.

Feminine hygiene pad 10 may include one or more main adhesive patch(es) 14, disposed about the longitudinal axis 200 of the pad, underlying the absorbent core and disposed on the garment/outward facing side of the backsheet 12. Main adhesive patch(es) 14 may be provided to enable the user to affix the pad to a suitable location within the underpants, such that the adhesive will cause the pad to remain in place during wear. Preferably, the main adhesive patch(es) 14 do not extend away from the longitudinal axis laterally beyond the longitudinal edges 15 of the absorbent core 13, to maintain a relatively uniform surface on the bottom on the pad for affixing to the underpants. The adhesive material may be selected so as to be suitable for securing the pad in place on the inner surface of the underpants material during wear conditions, but also to allow removal of the pad from the underpants following wear, without any substantial damage to the underpants material. The pad 10 may be supplied with a removable coversheet applied over the main adhesive patch(es) 14, to protect the patch surface(s) from contamination and prevent it (them) from premature/unwanted contact and adhesion with other surfaces prior to placement of the pad within the underpants for use. Alternatively, the pad may be supplied within a wrapper that is removably adhered to the main adhesive patch(es) 14, whereupon removal of the pad from the wrapper exposes to main adhesive patch(es) 14 for use.

Still referring to FIGS. 1 and 2A-2C, feminine hygiene pad 10 includes a pair of respective left and right side extensions 100. Each side extension 100 also includes a side extension adhesive patch 108. When so provided with a suitably-sized pad, and the pad 10 is applied to a suitable location within the user's underpants between the leg openings, the respective left and right side extensions each will extend laterally over an inside edge of a respective left or right leg opening in the underpants, and may be wrapped thereover and around toward the underside/outside surface of the underpants in the crotch region, between the leg openings. The side extension adhesive patch 108 may then be used to affix each extension 100 to the underside/outside surface of the underpants material. When so affixed, the side extensions 108 serve two purposes: First, by covering the edges of the underpants at the insides of the leg openings, they provide added protection against soiling of the underpants by exudates, about the inside portions of the leg openings. Second, the affixed side extensions provide an additional mechanism for holding the pad in place within the underpants, during wear. The pad 10 may be packaged and/or supplied with one or a plurality of removable coversheets applied over the side extension adhesive patches 108, to protect the patches' surfaces from contamination and prevent premature/unwanted contact and adhesion with other surfaces prior to placement of the pad within the underpants for use. In some examples, a single removable coversheet may be provided to cover all of the adhesive patches 108 and 14 included with the pad 10.

In some examples, side extensions 100 may be formed of integral, continuous extensions of one or more materials forming the topsheet 11 and/or backsheet 12. In some examples the side extensions 100 may be formed of integral, continuous extensions of each of the materials forming the topsheet 11 and backsheet 12, laminated together by adhesive, thermal bonding or a combination thereof. In other examples, side extensions 100 may be formed of discrete sections of one or more materials that have been affixed to the main structure of the pad along longitudinal sides thereof.

For purposes of wearer comfort as well as economy of materials usage in manufacture, the materials forming the side extensions 100 (e.g., topsheet and/or backsheet materials) may be relatively low-caliper, low basis weight materials. As a consequence, these materials may be quite thin and pliable. As such, they may be easily crumpled, or misfolded along lines other than most suitable for wrapping about the leg opening edges of the underpants. If this occurs when the side extension adhesive patches 108 are exposed, the side extensions may become stuck to themselves, or may be misplaced, compromising their utility and causing user frustration and dissatisfaction.

However, if a structure is provided that will promote linear folding or flexure along a single suitable fold or flexure location, and help prevent crumpling, the utility of the side extensions may be preserved and/or improved. Still referring to FIGS. 1 and 2A-2C, a supplementary structure 105 is included to each of the side extensions 100, thereby creating a supplemented zone 107 on the side extension. Supplementary structure 105 may be formed of any material that provides added basis weight and/or rigidity to the supplemented zone. Supplementary structure 105 may be formed of and/or include one or added layers of nonwoven web material, polymer film material, or even cellulose/pulp-based paper material, distinct from the layer(s) forming the side extension 100 in the longitudinal flexure zone 103 thereof. It may also be formed of and/or include an added or supplemental deposit of adhesive material, such as hot melt adhesive material otherwise used to laminate and hold other layers of the side extensions together. In this latter example, the supplemental adhesive material would be distinct from the adhesive material used to form the side extension adhesive patches 108, and would be disposed at a location within the side extension structure such that it is physically isolated from contact with external surfaces when the pad is in use (thus being differentiated from side extension adhesive patch 108 and main adhesive patch 14). A supplementary structure 105 may be disposed on the outside surface of the backsheet 12 material, or otherwise on the outward/garment-facing surface of the side extension 100 (as reflected in Fig. 2A), on the outside surface of the topsheet 11 material, or otherwise on the wearer-facing surface of the side extension 100 (as reflected in Fig. 2C), or between the topsheet 11 material and backsheet 12 material, or otherwise between wearing-facing and outward/garment-facing layers forming the side extension 100 (as reflected in Fig. 2B). Supplementary structure 105 may be affixed, adhered or bonded to, or between, the layer(s) forming the side extension 100 via adhesive such as hot melt adhesive, via a pattern of thermal bonds, mechanical bonds, thermal embossing, or a combination thereof.

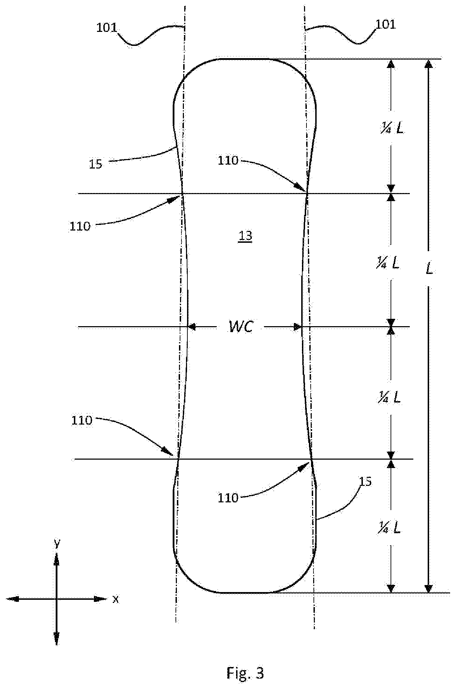

The following geometric references are identified and used herein as follows: The outboard-most portion of each side extension is outboard zone 104. Outboard zone 104 is the portion of the side extension that lies laterally outboard of longitudinal flexure zone 103. The outboardmost extent of longitudinal flexure zone 103 is demarcated by inboard supplementary structure tangent line 102, which lies tangent to supplementary structure 105 on the inboardmost edge thereof, and parallel to the nearest ¼ length chord line 101. Referring to Fig. 3, left and right ¼ length chord lines 101 are identified by dividing the length L of the absorbent core 13 equally into four equal sub-lengths ¼ L, and identifying intersections 110 between the lateral one-quarter and three-quarter length-dividing lines and the respective left and right longitudinal edges of the absorbent core 13. Each of the left and right ¼ length chord lines 101 is the line proximate the left or right longitudinal edge of the absorbent core, that connect the intersections 110 on that edge. (Identification of the ¼ length chord lines in this manner provides for suitable demarcation of inboardmost extents of longitudinal flexure zones 103, for varied shapes of absorbent cores. It will be appreciated that the left and right ¼ length chord lines may be, but are not necessarily, parallel to each other - depending upon the shape of the absorbent core.) Thus, referring again to Fig. 1, the longitudinal flexure zone 103 for each of the left and right sides is the zone between the ¼ length chord line 101 on that side, and the proximate inboard supplementary structure tangent line 102. Correspondingly, the outboard zone 104 of each side extension is that portion of the side extension lying laterally outboard of the proximate inboard supplementary structure tangent line 102.

The supplementary structure 105 (and resulting supplemented zone 107) may be suitably sized and located so as to promote formation of an orderly, linear fold or flexure of the side extension 100 substantially along the longitudinal direction, within longitudinal flexure zone 103. (For purposes herein, the supplemented zone 107 is deemed to be coextensive in plan profile, plan dimensions and plan surface area, with those of the supplementary structure 105.)

Simultaneously, it may desirable to prevent crumpling and/or unwanted folding of the side extensions 100 along lines or areas other than longitudinal flexure zones 103. Further, the longitudinal flexure zone 103 should be wide enough to provide a sufficient area of relatively highly flexible material to wrap about the leg opening edge of the underpants, including any elastic and/or trim materials forming a leg band in the underpants, to accommodate the curvature of the leg opening edge, and to accommodate a range of underpants leg opening spacings. Accordingly, it may be desired that each longitudinal flexure zone 103 be at least 3 mm, more preferably at least 4 mm, and even more preferably at least 5 mm wide, but no greater than 15 mm, more preferably no greater than 12mm, and even more preferably no greater than 10 mm wide, where its width is the distance between lines 101 and 102. For similar reasons it may be desired that the inboard supplementary structure tangent line (demarcating the inboardmost extent of the supplementary structure 105) be disposed entirely laterally outboard of the nearest left or right longitudinal side edge 15 of the absorbent core 13, in x-direction. To preserve the effects of the structure on promoting folding or flexure consistently through the longitudinal flexure zone 103, it may be preferred that the longitudinal flexure zone 103 have a substantially uniform structure and/or have a substantially uniform basis weight throughout. For purposes of promoting flexibility, the material(s) forming the longitudinal flexure zone 103 have a basis weight that is limited, to a quantity that is less than that of the supplemented zone 107. The basis weight of the supplemented zone 107 is at least 10 percent, more preferably at least 15 percent, still more preferably at least 20 percent, and even more preferably at least 25 percent, greater than the basis weight of the longitudinal flexure zone 103. In combination with such relationship, it may be further desired that the basis weight of the longitudinal flexure zone be no greater than 75 gsm, more preferably no greater than 70 gsm, and even more preferably no greater than 65 gsm.

To help prevent crumpling and/or folding in unsuitable areas, it may be desired that the supplementary structure 105 and resulting supplemented zone 107 occupy at least 60 percent, more preferably at least 75 percent, and even more preferably at least 85 percent, of the plan surface area of outboard zone 104. For similar reasons it may be desired that the supplemented zone 107 of each side extension be continuous and singular, rather than, *e.g.*, divided into more than one discrete sub-region by the presence of more than one discrete supplementary structure 105.

It may be desired that the side extensions 100 be limited in overall lateral width. This may be desired to further reduce the likelihood of crumpling, folding or flexing along unwanted locations. Additionally, it may be deemed desirable that the side extensions do not substantially overlap and/or adhere to each other beneath the wearer's underpants when the pad is applied therewithin, which may complicate removal from the underpants following use. Accordingly, it may be desired that the outboard zone 104 of each side extension 100 have a width WS no greater than one-half the width *WC* of the absorbent core 13, where the width *WC* of the absorbent core is measured along the x-direction at the length midpoint (*see* Fig. 3); and where the width WS of the outboard zone 104 of each side extension is measured along the x-direction and is the distance between the inboard supplementary structure tangent line 102 and the outermost edge of the side extension, along the lateral axis 201 (see FIG. 1).

Another feature that may be incorporated to promote folding or flexing only along the longitudinal flexure zone 103, and discourage folding or flexing in outboard zone 104, may be an appropriate aspect ratio for the supplementary structure 105 and resulting supplemented zone 107. In this regards, it may be desired that the supplementary structure have an aspect ratio of length (measured in the y-direction) to width (measured in the x-direction) greater than 1.0, more preferably at least 1.5, and more at least 2.0.

In order to help prevent flexure or formation of a fold between supplemented zone 107 and the portion of the side extension bearing the side extension adhesive patch 108, the surface area of the side extension adhesive patch 108 is disposed entirely within the supplemented zone 107.

Although, as explained above, providing a supplementary structure 105 to increase basis weight and optionally the rigidity of a supplemented zone 107 may provide the benefits described, the supplemented zone 107 should not be so rigid and/or bulky as to become a potential source of wearer discomfort. The side extensions 100 should be flexible enough to comfortably accommodate curvature about the wearer's body, and comfortably accommodate wearer movement, during wear/use of the pad 10, and should not be perceived during wear as a localized bulk beneath the underpants.

Accordingly, it may be desired that the supplemented zone 107, while having a basis weight greater than that of the longitudinal flexure zone 103, have a basis weight no greater than 125 gsm (grams per square meter), more preferably no greater than 100 gsm, and even more preferably no greater than 80 gsm. It may be desired that the supplemented zone 107, while having a flexural rigidity greater than that of the longitudinal flexure zone 103, has a flexural rigidity no greater than 200 $\mu$N/m, more preferably no greater than 150 $\mu$N/m, and even more preferably no greater than 100 $\mu$N/m, when measured using the Flexural Rigidity Measurement Method set forth below.

In some examples, the pad 10 may be provided with a visible indicium that readily visibly demarcates the outboard zones 104 of the side extensions 100. This can serve to provide an intuitive signal to the user/consumer that the side extensions 100 may be easily wrapped over the inside leg edges of the underpants, further promoting neat and wrapping thereabout as designed, and avoiding folding or wrapping along unsuitable areas. In one example, the supplemented zone 107 may be imparted with a color that visually contrasts with the coloring of the longitudinal flexure zone. The color may be imparted via, *e.g.*, printing, or by tinting or pigmenting materials used to form the layer components of the supplemented zone 107. In a more particular example, the supplementary structure 105 may be imparted with the visually contrasting color, thereby imparting the contrasting color to the supplemented zone. (Herein, a visually contrasting color is identified according the Visual Contrast method set forth below. In another example, the side extensions 100 and/or layer(s) thereof, and/or the longitudinal flexure zones 103, may each be imprinted with one or more visible lines extending along the longitudinal direction, thereby suggesting a line or longitudinally extending zone along which folding and/or flexibly wrapping are suitable.

Providing supplementary structure to a side extension including an adhesive patch as described herein has the effects of creating natural longitudinal flexure zone about which the side extension can reliably, easily and neatly flex and/or fold in a manner well-suited for wrapping about a proximate leg opening edge of the user's underpants. The supplementary structure can also add rigidity to the outboard and adhesive portions of the side extension. These effects substantially reduce chances that the side extension will be mis-folded, mis-applied, crumpled or bunched, or stuck to itself, during unpackaging and applying the product within the underpants. As a result, consumer/user satisfaction with the pad product may be improved, and the manufacturer and/or seller thereof may accordingly realize competitive advantage.

## Flexural Rigidity Measurement Method

Flexural rigidity of a sample of material taken from the longitudinal flexure zone and/or from the supplemented zone may be tested according to a method described in B. P. Saville, Physical Testing of Textiles (1999) (Section 10.1.3 - Hanging loop method), using the "pear" loop technique. Sample size may be selected as appropriate according to the dimensions of the longitudinal flexure zone and supplemented zone of the subject product.

## Visual Contrast

The color difference measurement is based on the CIE L*a*b* color system (CIELAB). A flat bed scanner capable of scanning a minimum of 24 bit color at 1200 dpi and has manual control of color management (a suitable scanner is an Epson Perfection V750 Pro from Epson America Inc., Long Beach CA) is used to acquire images. The scanner is calibrated against a color reflection target compliant to ANSI method IT8.7/2-1993 using color management software (a suitable package is MonacoEZColor available from X-Rite Grand Rapids, MI) to construct a scanner profile. The resulting calibrated scanner profile is opened within an imaging program that supports sampling in CIE L*a*b* (a suitable program is Photoshop S4 available from Adobe Systems Inc., San Jose, CA) to measure bonded and unbonded areas.

Turn on the scanner for 30 minutes prior to calibration. Place the IT8 target face down onto the scanner glass and close the scanner lid. Open the MonacoEZColor software and select acquire image using the Twain software included with the scanner. Within the Twain software deselect the unsharp mask setting and any automatic color correction or color management options that may be included in the software. If the automatic color management cannot be disabled, the scanner is not appropriate for this application. Acquire a preview scan at 200 dpi and 24 bit color. Insure that the scanned image is straight and first outer surface facing side-up. Crop the image to the edge of the target, excluding all white space around the target, and acquire the final image. The MonacoEZColor software uses this image to compare with included reference files to create and export a calibrated color profile compatible with Photoshop. After the profile is created the scan resolution (dpi) can be changed, but all other settings must be kept constant while imaging samples.

Provide respective same-sized, rectangular samples of each subject portion of the product, *i.e.*, the supplemented zone and the longitudinal flexure zone, of the largest length and width that are available. Precondition the samples at about 23°C $\pm$ 2 C° and about 50 % $\pm$ 2 % relative humidity for 2 hours prior to testing.

Open the scanner lid and place the first sample onto the scanner glass with the first outer surface facing the glass. Cover the sample with the white background (in this test method white is defined as having L* > 94, -2 < a* < 2, and -2 < b* < 2) and close the lid. Acquire and import a scan of the first sample into Photoshop at 600 dpi and 24 bit color. Assign the calibrated scanner profile to the image and change the mode to Lab Color ("Lab Color" in Photoshop corresponds to the CIE L* a* b* standard). Select the "eyedropper" color selection tool. Set the sampling size of the tool to include as many pixels as possible within an area of the sample 2mm by 2mm square. Using the eyedropper tool measure and record L*a*b* values in 10 different 2mm by 2mm square areas (not having apertures) in the sample image. Average the 10 individual L*a*b* values and record as $L_1$, $a_1$, and $b_1$ respectively.

Repeat the steps in the paragraph above for the second sample, and record the averaged values as $L_2$, $a_2$ and

b$_2$. Calculate and report the color difference (delta E*) between the bonded and unbonded areas using the following equation:

$$\text{delta } E^* = \sqrt{(L_2^* - L_1^*)^2 + (a_2^* - a_1^*)^2 + (b_2^* - b_1^*)^2}$$

and report to the nearest 0.01 units. A total of three substantially identical samples of each layer are measured for each sample set. Average the three delta E* values and report to the nearest 0.1 unit.

It is expected that the person of ordinary skill in the relevant art is capable of making any suitable adjustments in sampling and/or data collection necessary to the method, according to the dimensions of the subject product, to make a relevant comparison between the respective colors of the longitudinal flexure zone and of the supplemented zone, to determine whether the visual contrast specified herein is present.

While particular examples of the present invention have been illustrated and described, it would be obvious to those skilled in the art that various other changes and modifications may be made without departing from the scope of the invention. It is therefore intended to cover in the appended claims all such changes and modifications that are within the scope of this invention.

**Claims**

1. An absorbent feminine hygiene article (10), comprising a topsheet (11) comprising a liquid permeable topsheet web material, a backsheet (12) comprising a substantially liquid impermeable backsheet web material, and an absorbent core structure (13) disposed between the topsheet and backsheet, the absorbent core structure having oppositely-disposed left and right side edges (15) and associated respective left and right core ¼ length chord lines (101), wherein the ¼ length chord lines are identified by dividing the length L of the absorbent core structure (13) equally into four equal sub-lengths ¼ L, and identifying intersections (110) between the lateral one-quarter and three-quarter length-dividing lines and the respective left and right longitudinal edges of the absorbent core structure (13); the article also comprising a pair of oppositely-disposed left and right side extensions (100), each side extension extending laterally away from one of the side edges of the absorbent core structure, wherein:

    each side extension comprises an extension portion of web material that is either integral and continuous with one or both of the topsheet and backsheet web materials, or is directly or indirectly attached to at least one of the topsheet and backsheet web materials;
    each side extension comprises a longitudinal flexure zone (103) and an outboard zone (104), the outboard zone comprising a supplementary structure (105) having an inboard supplementary structure tangent line (102) parallel to a nearest of the left and right core ¼ length chord lines (101), the longitudinal flexure zone (103) being bounded at least in part by the inboard supplementary structure tangent line (102) and the nearest of the ¼ length chord lines (101); the outboard zone (104) being that portion of the side extension (100) lying outboard of the inboard supplementary structure tangent line (102); the outboard zone (104) comprising a supplemented zone (107) bounded by an outer plan profile of the supplementary structure (105);
    the longitudinal flexure zone (103) has a first basis weight, and the supplemented zone (107) has a second basis weight that is at least 10 percent, more preferably at least 15 percent, still more preferably at least 20 percent, and even more preferably at least 25 percent greater than the first basis weight; and
    the supplemented zone (107) has a side extension adhesive patch (108) disposed at least in part thereon, the side extension adhesive patch (108) being distinct from the supplementary structure (105),
    wherein the side extension adhesive patch (108) is disposed entirely within the supplemented zone (107).

2. The article of claim 1 wherein, in each side extension, the supplemented zone (107) occupies at least 60 percent, more preferably at least 75 percent, and even more preferably at least 85 percent, of a plan surface area of the outboard zone.

3. The article of either of the preceding claims wherein, in each side extension, the supplemented zone (107) is continuous.

4. The article of any of the preceding claims wherein the supplementary structure (105) comprises a layer of material distinct from the extension portion of web material.

5. The article of claim 4 wherein the supplementary structure (105) comprises a layer of nonwoven web material.

6. The article of either of claims 4 or 5 wherein the supplementary structure (105) comprises a layer of film.

7. The article of any of the preceding claims wherein the supplementary structure (105) comprises a pattern of thermal bonds and/or a pattern of thermal embossing.

8. The article of any of the preceding claims wherein the supplementary structure (105) comprises a supplementary deposit of isolated adhesive material.

9. The article of any of the preceding claims wherein the supplemented zone (107) has a flexural rigidity no greater than 200 $\mu$N/m.

10. The article of any of the preceding claims wherein the inboard supplementary structure tangent line (102) of each side extension is disposed entirely laterally outboard a nearest of the left and right side edges of the absorbent core structure.

11. The article of any of the preceding claims wherein the longitudinal flexure zone (103) has a substantially uniform basis weight throughout.

12. The article of any of the preceding claims wherein the longitudinal flexure zone (103) is at least 3 mm, more preferably at least 4 mm, and even more preferably at least 5 mm wide, measured along a direction perpendicular to the inboard supplementary structure tangent line (102).

13. The article of any of the preceding claims wherein the longitudinal flexure zone (103) is no greater than 15 mm, more preferably no greater than 12 mm, and even more preferably no greater than 10 mm wide, measured along a direction perpendicular to the inboard supplementary structure tangent line (102).

14. The article of any of the preceding claims wherein the absorbent core (13) has a first width WC, the outboard zones (104) of the side extensions (100) each have second widths WS, and WS of at least one and preferably both side extensions is no more than one-half WC.


**Patentansprüche**

1. Absorbierender Damenhygieneartikel (10), umfassend: eine Oberschicht (11), die ein flüssigkeitsdurchlässiges Oberschicht-Bahnmaterial umfasst, eine Unterschicht (12), die ein im Wesentlichen flüssigkeitsundurchlässiges Unterschicht-Bahnmaterial umfasst, und eine Absorptionskernstruktur (13), die zwischen der Oberschicht und der Unterschicht angeordnet ist, wobei die Absorptionskernstruktur einen linken und einen rechten Seitenrand (15) und jeweils eine zugehörige linke bzw. rechte ¼-Längen-Sehnenlinie des Kerns (101) aufweist, wobei die ¼-Längen-Sehnenlinien durch gleichmäßiges Teilen der Länge L der Absorptionskernstruktur (13) in vier gleiche Unterlängen ¼ L und Identifizieren von Schnittpunkten (110) zwischen der lateralen Einviertel- und Dreiviertel-Längenteilungslinie und dem linken bzw. dem rechten Längsrand der Absorptionskernstruktur (13) identifiziert werden; wobei der Artikel auch ein Paar aus einer linken und einer rechten Seitenerweiterung (100), die einander entgegengesetzt angeordnet sind, umfasst, wobei sich jede Seitenerweiterung seitlich von einem der Seitenränder der Absorptionskernstruktur weg erstreckt, wobei:

jede Seitenerweiterung einen Erweiterungsabschnitt aus Bahnmaterial umfasst, der entweder einstückig und fortlaufend mit einem oder beiden von dem Oberschicht- und dem Unterschicht-Bahnmaterial ist oder direkt oder indirekt an mindestens einem von dem Oberschicht- und dem Unterschicht-Bahnmaterial angebracht ist; wobei jede Seitenerweiterung eine Längsbiegezone (103) und eine Außenzone (104) umfasst, wobei die Außenzone eine Ergänzungsstruktur (105) umfasst, die eine Ergänzungsstruktur-Innentangente (102) parallel zu einer nächstgelegenen von der linken und der rechten ¼-Längen-Sehnenlinie des Kerns (101) aufweist, wobei die Längsbiegezone (103) zumindest teilweise durch die Ergänzungsstruktur-Innentangente (102) und die nächstgelegene von den ¼-Längen-Sehnenlinien (101) begrenzt ist; wobei die Außenzone (104) der Abschnitt der Seitenerweiterung (100) ist, der außerhalb der Ergänzungsstruktur-Innentangente (102) liegt; wobei die Außenzone (104) eine ergänzte Zone (107) umfasst, die durch ein äußeres Planprofil der Ergänzungsstruktur (105) begrenzt ist;
die Längsbiegezone (103) ein erstes Basisgewicht aufweist und die ergänzte Zone (107) ein zweites Basisgewicht aufweist, das mindestens 10 Prozent, mehr bevorzugt mindestens 15 Prozent, noch mehr bevorzugt mindestens 20 Prozent und noch mehr bevorzugt mindestens 25 Prozent größer ist als das erste Basisgewicht; und

die ergänzte Zone (107) ein Seitenerweiterungs-Klebstofffeld (108) aufweist, das zumindest teilweise darauf angeordnet ist, wobei sich das Seitenerweiterungs-Klebstofffeld (108) von der Ergänzungsstruktur (105) unterscheidet,

wobei das Seitenerweiterungs-Klebstofffeld (108) vollständig innerhalb der Ergänzungszone (107) angeordnet ist.

2. Artikel nach Anspruch 1, wobei in jeder Seitenerweiterung die ergänzte Zone (107) mindestens 60 Prozent, mehr bevorzugt mindestens 75 Prozent und noch mehr bevorzugt mindestens 85 Prozent eines Plan-Oberflächenbereichs der Außenzone einnimmt.

3. Artikel nach einem der vorstehenden Ansprüche, wobei in jeder Seitenerweiterung die ergänzte Zone (107) ununterbrochen ist.

4. Artikel nach einem der vorstehenden Ansprüche, wobei die Ergänzungsstruktur (105) eine Schicht aus Material umfasst, das sich von dem Erweiterungsabschnitt-Bahnmaterial unterscheidet.

5. Artikel nach Anspruch 4, wobei die Ergänzungsstruktur (105) eine Schicht aus Vliesbahnmaterial umfasst.

6. Artikel nach einem der Ansprüche 4 oder 5, wobei die Ergänzungsstruktur (105) eine Folienschicht umfasst.

7. Artikel nach einem der vorstehenden Ansprüche, wobei die Ergänzungsstruktur (105) ein Muster von thermischen Bindungen und/oder ein Muster von thermischem Prägen umfasst.

8. Artikel nach einem der vorstehenden Ansprüche, wobei die Ergänzungsstruktur (105) eine Ergänzungsanordnung von isoliertem Klebstoffmaterial umfasst.

9. Artikel nach einem der vorstehenden Ansprüche, wobei die ergänzte Zone (107) eine Biegesteifigkeit von nicht mehr als 200 $\mu$N/m aufweist.

10. Artikel nach einem der vorstehenden Ansprüche, wobei die Ergänzungsstruktur-Innentangente (102) jeder Seitenerweiterung lateral vollständig außerhalb eines nächstgelegenen von dem linken und dem rechten Seitenrand der Absorptionskernstruktur angeordnet ist.

11. Artikel nach einem der vorstehenden Ansprüche, wobei die Längsbiegezone (103) durchgehend ein im Wesentlichen gleichmäßiges Basisgewicht aufweist.

12. Artikel nach einem der vorstehenden Ansprüche, wobei die Längsbiegezone (103) mindestens 3 mm, mehr bevorzugt mindestens 4 mm und noch mehr bevorzugt mindestens 5 mm breit ist, gemessen entlang einer Richtung senkrecht zur Ergänzungsstruktur-Innentangente (102).

13. Artikel nach einem der vorstehenden Ansprüche, wobei die Längsbiegezone (103) nicht mehr als 15 mm, mehr bevorzugt nicht mehr als 12 mm und noch mehr bevorzugt nicht mehr als 10 mm breit ist, gemessen entlang einer Richtung senkrecht zur Ergänzungsstruktur-Innentangente (102).

14. Artikel nach einem der vorstehenden Ansprüche, wobei der Absorptionskern (13) eine erste Breite WC aufweist, die Außenzonen (104) der Seitenerweiterungen (100) jeweils zweite Breiten WS aufweisen, und WS von mindestens einer und vorzugsweise beiden Seitenerweiterungen nicht mehr als die Hälfte von WC beträgt.

## Revendications

1. Article absorbant d'hygiène féminine (10), comprenant une feuille de dessus (11) comprenant un matériau de nappe de feuille de dessus perméable aux liquides, une feuille de fond (12) comprenant un matériau de nappe de feuille de fond essentiellement imperméable aux liquides, et une structure d'âme absorbante (13) disposée entre la feuille de dessus et la feuille de fond, la structure d'âme absorbante ayant des bords latéraux gauche et droit disposés de façon opposée (15) et associés à des lignes de corde de quart de longueur d'âme gauche et droite respectives (101), dans lequel les lignes de corde de quart de longueur sont identifiées en divisant la longueur L de la structure d'âme absorbante (13) de manière égale en quatre sous-longueurs égales valant ¼ de L, et en identifiant des intersections (110) entre les lignes latérales de division à un quart et trois-quarts de la longueur et les bords longitudinaux gauche et droit respectifs de la structure d'âme absorbante (13) ; l'article comprenant également une paire d'extensions latérales gauche et droite disposées de façon opposée (100), chaque extension latérale s'étendant latéralement à l'écart d'un des bords latéraux de la structure d'âme absorbante, dans lequel :

chaque extension latérale comprend une partie d'extension de matériau de nappe qui est soit solidaire et continue avec l'un et/ou l'autre des matériaux de nappe de feuille de dessus et de feuille de fond, soit est fixée directement ou indirectement à au moins l'un des matériaux de nappe de feuille de dessus et de feuille de fond ; chaque extension latérale comprend une zone de flexion longitudinale (103) et une zone extérieure (104), la zone extérieure comprenant une structure complémentaire (105) ayant une ligne tangente de structure complémentaire intérieure (102) parallèle à une plus proche parmi les lignes de corde de quart de longueur d'âme gauche et droite (101), la zone de flexion longitudinale (103) étant délimitée au moins en partie par la ligne tangente de structure complémentaire intérieure (102) et la plus proche parmi les lignes de corde de quart de longueur (101) ; la zone extérieure (104) étant cette partie de l'extension latérale (100) se trouvant à l'extérieur de la ligne tangente de structure complémentaire intérieure (102) ; la zone extérieure (104) comprenant une zone complétée (107) délimitée par un profil de plan externe de la structure complémentaire (105) ; la zone de flexion longitudinale (103) a une première masse surfacique, et la zone complétée (107) a une deuxième masse surfacique qui est au moins 10 pour cent, plus préférablement au moins 15 pour cent, encore plus préférablement au moins 20 pour cent, et même plus préférablement au moins 25 pour cent plus grande que la première masse surfacique ; et la zone complétée (107) a une pastille adhésive d'extension latérale (108) disposée au moins en partie sur celle-ci, la pastille adhésive d'extension latérale (108) étant distincte de la structure complémentaire (105), dans lequel la pastille adhésive d'extension latérale (108) est disposée entièrement à l'intérieur de la zone complétée (107).

2. Article selon la revendication 1, dans lequel, dans chaque extension latérale, la zone complétée (107) occupe au moins 60 pour cent, plus préférablement au moins 75 pour cent, et encore plus préférablement au moins 85 pour cent, d'une superficie en plan de la zone extérieure.

3. Article selon l'une ou l'autre des revendications précédentes dans lequel, dans chaque extension latérale, la zone complétée (107) est continue.

4. Article selon l'une quelconque des revendications précédentes dans lequel la structure complémentaire (105) comprend une couche de matériau distincte de la partie d'extension de matériau de nappe.

5. Article selon la revendication 4 dans lequel la structure complémentaire (105) comprend une couche de matériau de nappe non tissée.

6. Article selon l'une ou l'autre des revendications 4 ou 5 dans lequel la structure complémentaire (105) comprend une couche de film.

7. Article selon l'une quelconque des revendications précédentes dans lequel la structure complémentaire (105) comprend un motif de liaisons thermiques et/ou un motif de gaufrages thermiques.

8. Article selon l'une quelconque des revendications précédentes dans lequel la structure complémentaire (105) comprend un dépôt complémentaire de matériau adhésif isolé.

9. Article selon l'une quelconque des revendications précédentes dans lequel la zone complétée (107) a une rigidité en flexion n'excédant pas 200 $\mu$N/m.

10. Article selon l'une quelconque des revendications précédentes dans lequel la ligne tangente de structure complémentaire intérieure (102) de chaque extension latérale est entièrement disposée latéralement à l'extérieur d'un plus proche parmi les bords latéraux gauche et droit de la structure d'âme absorbante.

11. Article selon l'une quelconque des revendications précédentes dans lequel la zone de flexion longitudinale (103) a une masse surfacique essentiellement uniforme partout.

**12.** Article selon l'une quelconque des revendications précédentes dans lequel la zone de flexion longitudinale (103) a une largeur d'au moins 3 mm, plus préférablement d'au moins 4 mm, et encore plus préférablement d'au moins 5 mm, mesurée le long d'une direction perpendiculaire à la ligne tangente de structure complémentaire intérieure (102).

**13.** Article selon l'une quelconque des revendications précédentes dans lequel la zone de flexion longitudinale (103) a une largeur qui n'est pas supérieure à 15 mm, plus préférablement pas supérieure à 12 mm, et encore plus préférablement pas supérieure à 10 mm, mesurée le long d'une direction perpendiculaire à la ligne tangente de structure complémentaire intérieure (102).

**14.** Article selon l'une quelconque des revendications précédentes dans lequel l'âme absorbante (13) a une première largeur WC, les zones extérieures (104) des extensions latérales (100) ont chacune des deuxièmes largeurs WS, et la WS d'au moins une et de préférence de l'une et l'autre des extensions latérales n'est pas supérieure à la moitié de WC.

Fig. 1

10

13

103    11    103

100                    100

108
105    12    14    105    108

**Fig. 2A**

z
x

10

13

103    11    103

100                    100

108
105    12    14    105    108

**Fig. 2B**

z
x

10

13

105    103    11    103    105

100                    100

108
12    14    108

**Fig. 2C**

z
x

Fig. 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2016213526 A1
- US 5550167 A
- US 5387207 A
- US 5352711 A
- US 5331015 A
- US 5843063 A
- US 5879343 A

**Non-patent literature cited in the description**

- **B. P. SAVILLE.** *Physical Testing of Textiles,* 1999